# EUROPEAN PATENT SPECIFICATION

(11) **EP 2 192 409 B1**
(45) Date of publication and mention of the grant of the patent: **23.04.2014**
(21) Application number: 08305845.3
(22) Date of filing: 26.11.2008
(51) Int. Cl.: G01N 33/543

(54) **Label independent detection biosensor composition and methods thereof**
Biosensorzusammensetzung zur Marker-unabhängigen Erkennung und Verfahren dafür
Composition de biocapteur pour la détection indépendante de marqueurs et procédés correspondants

(43) Date of publication of application: 02.06.2010
(73) Proprietor: Corning Incorporated, Corning NY 14831 (US)
(72) Inventor: Deshayes, Sophie, 77515 Faremoutiers (FR); Henry, David, 91150 Morigny-Champigny (FR)
(74) Representative: Chantraine, Sylvie Hélène

(56) References cited:
- EP-A2- 0 226 470
- WO-A-2004/076511
- WO-A-2006/058237
- JP-A- 2008 002 918
- US-A- 4 235 867
- US-A1- 2007 111 273
- BRUEGGEMEIER S B ET AL: "Use of protein-acrylamide copolymer hydrogels for measuring protein concentration and activity" ANALYTICAL BIOCHEMISTRY, vol. 329, no. 2, 2004, pages 180-189, XP004509813
- DATABASE WPI Week 200477 Thomson Scientific, London, GB; AN 2004-777557 XP002524461 & JP 2004 301528 A (SEKISUI CHEM IND CO LTD) 28 October 2004 (2004-10-28)
- DATABASE WPI Week 200477 Thomson Scientific, London, GB; AN 2004-777559 XP002524462 & JP 2004 301530 A (SEKISUI CHEM IND CO LTD) 28 October 2004 (2004-10-28)

## Description

### BACKGROUND

The disclosure generally relates to biosensors for label independent detection (LID), and more particularly to surface chemistry for a high efficiency resonant grating biosensor, such as Epic^{®} biosensors, and to methods of preparation and use.

### SUMMARY

The disclosure provides surface treated biosensor articles for label independent detection (LID), and methods for their preparation and use. The biosensors have high immobilizing and binding efficiency, and like properties, in resonant grating and like sensing applications. The disclosure also concerns systems and methods providing sensors capable of immobilizing bioentities (e.g., receptor proteins, and like cellular targets) at high density and providing superior sensitivity with respect to detecting an analyte (e.g., ligand) than previously reported. The LID biosensors of the disclosure have high sensitivity for the detection or recognition of bio-molecular events. The treated biosensor surfaces of the disclosure can exhibit increased ligand binding, consume less protein, and provide greater sensitivity compared to known treated biosensor surfaces. The treated biosensor surfaces of the disclosure are also suitable for cell culture.

### BRIEF DESCRIPTION OF THE DRAWINGS

Fig. 1 is a schematic summary of the steps carried out to form the polymer *in situ* on the surface of the LID biosensor, in embodiments of the disclosure.

Figs. 2 and 3 show a graph of carbonic anhydrase II (CAII) immobilization response measured with an Epic^{®} instrument on biosensors having the disclosed and the comparative surface chemistries, in embodiments of the disclosure.

Fig. 4 shows a graph of carbonic anhydrase (II) availability (%) as a function of concentration for the disclosed and comparative surface chemistries, in embodiments of the disclosure

Fig. 5 shows a graph of furosemide binding response measured with an Epic^{®} instrument on biosensors having the disclosed and the comparative surface chemistries, in embodiments of the disclosure.

Fig. 6 shows a graph of the influence of acrylic acid/acrylamide ratios for the disclosed surfaces on the furosemide binding response, in embodiments of the disclosure.

Fig. 7 shows a graph of streptavidine (SA) immobilization response for the disclosed and the comparative surfaces measured with an Epic^{®} instrument, in embodiments of the disclosure.

Figs. 8 and 9 show graphs of biotin binding response and availability for the disclosed and the comparative surfaces measured with an Epic^{®} instrument, in embodiments of the disclosure.

Fig. 10 shows a photograph illustrative of the non-uniformity of CAII immobilization in a 384-well plate having a comparative surface using the Epic^{®} 2 D Map software, in embodiments of the disclosure.

Fig. 11 shows a photograph illustrative of the uniformity of CAII immobilization in a 384-well plate having a disclosed surface using the Epic^{®} 2 D Map software, in embodiments of the disclosure.

Fig. 12 is a schematic of illustrative surface polymerized polymer structures, in embodiments of the disclosure.

Fig. 13 is a schematic of illustrative activated polymer structures, in embodiments of the disclosure.

### DETAILED DESCRIPTION

Various embodiments of the disclosure will be described in detail with reference to drawings, if any. Reference to various embodiments does not limit the scope of the invention, which is limited only by the scope of the claims attached hereto. Additionally, any examples set forth in this specification are not limiting and merely set forth some of the many possible embodiments for the claimed invention.

### Definitions

"Assay," "assaying," or like terms refers to an analysis to determine, for example, the presence, absence, quantity, extent, kinetics, dynamics, or type of a biologic's or cell's optical or bioimpedance response upon stimulation with an exogenous stimuli, such as a ligand candidate compound, a viral particle, a pathogen, a surface or culture condition, or like entity. Such terms can also include non-biologic or non-cell responses to stimuli or reactions to stimuli.

"Attach," "attachment," "adhere," "adhered," "adherent," "immobilized," or like terms generally refer to immobilizing or fixing, for example, a surface modifier substance, a compatibilizer, a cell, a ligand candidate compound, and like entities of the disclosure, to a surface, such as by physical absorption, chemical bonding, and like processes, or combinations thereof. Particularly, "cell attachment," "cell adhesion," or like terms refer to the interacting or binding of cells to a surface, such as by culturing, or interacting with a cell anchoring material, a compatibilizer (e.g., fibronectin, collagen, lamin, gelatin, polylysine, etc.), or like entity.

"Adherent cells" refers to a cell or a cell line or a cell system, such as a prokaryotic or eukaryotic cell, that remains associated with, immobilized on, or in certain contact with the outer surface of a substrate. Such type of cells after culturing can withstand or survive washing and medium exchanging process, a process that is prerequisite to many cell-based assays. "Weakly adherent cells" refers to a cell, a cell line, or a cell system, such as a prokaryotic or eukaryotic cell, which weakly interacts, associates with, or contacts the surface of a substrate during cell culture. However, these types of cells, for example, human embryonic kidney (HEK) cells, tend to dissociate easily from the surface of a substrate by physically disturbing approaches, such as washing or medium exchange. "Suspension cells" refer to a cell or a cell line that is preferably cultured in a medium wherein the cells do not attach or adhere to the surface of a substrate during the culture. "Cell culture" or "cell culturing" refers to the process by which either prokaryotic or eukaryotic cells are grown under controlled conditions. "Cell culture" can also refer to the culturing of cells derived from multicellular eukaryotes, especially animal cells, including the culturing of complex tissues and organs.

"Cell" or like term refers to a small usually microscopic mass of protoplasm bounded externally by a semipermeable membrane, optionally including one or more nuclei and various other organelles, capable alone or interacting with other like masses of performing all the fundamental functions of life, and forming the smallest structural unit of living matter capable of functioning independently including synthetic cell constructs, cell model systems, and like artificial cellular systems.

"Cell system" or like term refers to a collection of more than one type of cells (or differentiated forms of a single type of cell), which interact with each other, thus performing a biological or physiological or pathophysiological function. Such cell system include, for example, an organ, a tissue, a stem cell, a differentiated hepatocyte cell, or like systems.

"Stimulus," "therapeutic candidate compound," "therapeutic candidate," "prophylactic candidate," "prophylactic agent," "ligand candidate," "ligand," or like terms refer to a molecule or material, naturally occurring or synthetic, which is of interest for its potential to interact with a cell attached to the biosensor or a pathogen. A therapeutic or prophylactic candidate can include, for example, a chemical compound, a biological molecule, a peptide, a protein, a biological sample, a drug candidate small molecule having, for example, a molecular weight of less than about 1,000 Daltons, a drug candidate biologic molecule, a drug candidate small molecule-biologic conjugate, and like materials or molecular entity, or combinations thereof, which can specifically bind to or interact with at least one of a cellular target or a pathogen target such as a protein, DNA, RNA, an ion, a lipid, or like structure or component of a live-cell.

"Biosensor" or like terms generally refer to a device for the detection of an analyte that combines a biological component with a physicochemical detector component. The biosensor typically consists of three parts: a biological component or element (such as a cellular target, tissue, microorganism, pathogen, live-cell, or a combination thereof), a detector element (operating in a physicochemical manner such as optical, piezoelectric, electrochemical, thermometric, or magnetic), and a transducer associated with both components. In embodiments, an optical biosensor can comprise an optical transducer for converting a molecular recognition or molecular stimulation event in a cellular target, a live-cell, a pathogen, or a combination thereof into a quantifiable signal.

"Include," "includes," or like terms refers to including but not limited to, that is, inclusive and not exclusive.

"About" modifying, for example, the quantity of an ingredient in a composition, concentrations, volumes, process temperature, process time, yields, flow rates, pressures, and like values, and ranges thereof, employed in describing the embodiments of the disclosure, refers to variation in the numerical quantity that can occur, for example: through typical measuring and handling procedures used for making compounds, compositions, concentrates, or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture. The claims appended hereto are intended to include equivalents of these quantities with or without the "about" modifier.

"Optional," "optionally," or like terms refer to the subsequently described event or circumstance can or cannot occur, and that the description includes instances where the event or circumstance occurs and instances where it does not. For example, the phrase "optional component" means that the component can or cannot be present and that the disclosure includes both embodiments including and excluding the component.

"Consisting essentially of" in embodiments refers, for example, to a surface composition, a method of making or using a surface composition, formulation, or composition on the surface of the biosensor, and articles, devices, or apparatus of the disclosure, and can include the components or steps listed in the claim, plus other components or steps that do not materially affect the basic and novel properties of the compositions, articles, apparatus, and methods of making and use of the disclosure, such as particular reactants, particular additives or ingredients, a particular agents, a particular cell or cell line, a particular surface modifier or condition, a particular ligand candidate, or like structure, material, or process variable selected. Items that may materially affect the basic properties of the components or steps of the disclosure or may impart undesirable characteristics to the present disclosure include, for example, thick layers of the polymeric surface layer, such as greater than about 300 to about 500 nm, and like considerations.

The indefinite article "a" or "an" and its corresponding definite article "the" as used herein means at least one, or one or more, unless specified otherwise.

Abbreviations, which are well known to one of ordinary skill in the art, may be used, for example, "h" or "hr" for hour or hours, "g" or "gm" for gram(s), "mL" for milliliters, "RT" for room temperature, "nm" for nanometers, and like abbreviations.

"Weight percent," "wt%," "percent by weight," or like terms with reference to, for example, a component, unless specifically stated to the contrary, refer to the ratio of the weight of the component to the total weight of the composition in which the component is included, expressed as a percentage.

Specific and preferred values disclosed for components, ingredients, additives, cell types, antibodies, and like aspects, and ranges thereof, are for illustration only; they do not exclude other defined values or other values within defined ranges. The compositions, apparatus, and methods of the disclosure include those having any value or any combination of the values, specific values, more specific values, and preferred values described herein.

The issue of low target immobilization on a biosensor and low specific ligand binding activity on a biosensor can be overcome by having the thin film functionalized surfaces of the disclosure having high biochemical target immobilization on a biosensor and high specific ligand binding activity with the surface-bound biochemical target or like compliment.

The LID method can be based on the local change of refractive index induced by, for example, the adsorption of a ligand onto an immobilized target such as a receptor(s).

The surface chemistry of the disclosure enhances the resulting signal by increasing the immobilized receptor density, i.e., the number of the receptors immobilized on the biosensor which in turn provides increased capture capacity for targeted ligands, and by providing increased activity or availability of the immobilized receptor.

The surface chemistry of the disclosure may be compatible with Dual Polarized Intereferometry (DPI), which is another type of LID sensor, or surface plasmon resonance (SPR) type sensors.

Polymer gels that have been previously described which can immobilize biomolecules on LID biosensors that are made of, for example, a polysaccharide, such as dextran, or a synthetic polymer, such as polyacrylic acid, see, for example, U.S. Patent No. 5,436,161, assigned to BIAcore.

Among the numerous types of polymer gels, one frequently used is based on carboxymethyl dextran as described, for example, in S. Lofas, et al., "A Novel Hydrogel Matrix on Gold Surfaces in Surface Plasmon Resonance Sensors for Fast and Efficient Covalent Immobilization of Ligands," J. Chem. Soc., Chem. Commun., 1990, 21, 1526-1528, and the aforementioned U.S. Patent No. 5,436,161. This carboxymethyl dextran based gel has limited capture capacity due to its molecular weight (M_{w} 500,000 g/mol for the CM5 sensor chip available from BIAcore) which makes the height or thickness of the final gel unsuitable for very high protein capture capacity.

A similar approach was described in PCT Publication No. WO 2007/049269, "Binding layer and method for its preparation and uses thereof," S. Nimri (Applicant Bio-Rad). This publication mentions binding layers comprising a polysaccharide substituted with carboxylic acid groups exhibiting high performance in the binding of ligand molecules and in the interaction with analyte molecules. The polysaccharide is modified by reaction with an alanine spacer. This publication also mentions that the spacer modification allows more efficient activation of the carboxylic acid groups of the spacer compared to activation of the carboxylic acid groups of a known carboxymethylated polysaccharide. This publication further mentions that synthetic polymers, like poly(acrylic acid) or poly(methacrylic acid), exhibit much more efficient activation and subsequent immobilization. However, the ligand molecules exhibited low activity perhaps due to lower "biocompatibility" of these polymers (see p.13, lines 5-9).

In addition to low protein activity, when "grafting-to" a poly(methacrylic acid) polymer, the density of polymer that has been grafted is limited and decreases the amount of protein captured. This low grafting density has been attributed to mainly steric hindrance and repulsive forces occurring between the polymer chains that try to reach the substrate. In addition, the use of a medium molecular weight polymer to get high polymer grafting density limits the thickness of the gel and the amount of protein captured. To overcome this drawback, *in-situ* synthesis of the polymer directly on the sensor surface has gained some interest. For example, sophisticated polymerization techniques, called "grafting-from," using controlled free-radical polymerization, such as RAFT or ATRP, can be also used to make thicker gels and gels having a high density of polymer chains grafted to a surface but such methods suffer from many drawbacks. For example, these in-situ polymerization methods can be quite slow, may require specific anchored polymerization initiators, are complex to run, may have undesirable initiator residues remaining in the polymer gel layer, and may require long washing times to remove unbound materials. Moreover, although accurate control of the molecular weight of the polymer generated and a very high polymer grafting density was obtained by perfect tethering of only one end of the polymer chain, the length of the polymer chain obtained was not very great for a given polymerization time frame and thus limits the thickness of the gel layer for biomolecule capture, see J. Brittain, et al., "A Facile Route to Poly (Acrylic Acid) Brushes Using Atom Transfer Radical Polymerization," Macromolecules, 2006, Vol. 39, pgs. 26-29.

Another method disclosed in U.S. Patent No. 7,250,253, entitled "Immobilization of molecules on surfaces via polymer brushes," Klapproth, et al., involves immobilizing a monolayer of radical polymerization initiator but using uncontrolled free radical polymerization. The molecular weight of the polymer may be as high as, e.g., 100,000 g/mol, but this value remains even lower than the 500,000 g/mol obtained with the grafting-to technique using the T500 carboxymethyldextran mentioned in U.S. Patent No. 5,436,161. Another drawback of the '253 patent method is the synthesis of a specific initiator anchored on the substrate. A major limitation comes from the long extraction time required to eliminate un-reacted monomers and non-bonded polymer.

When LID biosensors such as grating resonant sensors or SPR-sensors are used, the evanescent field wave is only able to probe the first 100 to 200 nanometers from the surface into the media to detect molecular recognition events. Thus, micrometers thick gels are inoperable and unsuitable as surface chemistry for LID techniques because the diffusion of proteins to be immobilized is very slow and only a small amount of protein reaches the probed region. Additionally, most of the binding of the small molecules (e.g., drugs) to much of the immobilized protein(s) occurs beyond the probed region and thus a recognition event or binding cannot be detected.

A device and preparative process are described in EP 0,226,470 patent (the "'470 patent") entitled "Materials and methods for microchemical testing," J.A.
Bosley, et al., (assigned to Unilever). However, only methods for preparing thick gels are described. The working examples demonstrate gel thickness having multi-micrometer thicknesses. The '470 patent mentions that smoothness of the resulting gel surface can be obtained by clamping a flat non-stick solid surface over the coated substrate. Unfortunately, even when high clamping pressure was applied to the coated substrate and the non-stick surface assembly, we discovered that suitably thin gel layers, such as less than about 200 nm thick and compatible with LID biosensors could not be obtained.

The '470 patent describes an apparatus for carrying out a microchemical test, comprising a solid substrate having a surface which carries a polymer hydrogel formed *in situ* thereon and covalently bonded thereto. The polymer gel is covalently bonded to the substrate using an organic trialkoxy silane compound (e.g., MOPS) conjugated with a mer (such as an acrylate) of the hydrogel polymer. Example polymer gels included those made by *in situ* polymerization of acrylic monomers, such as methacrylic acid and methacrylamide. The hydrogel depending on the buffer can be net negatively charged due to the presence of carboxylic acid groups provided by the methacrylic acid. The carboxylic acid groups contained in the polymer matrix can be activated by reacting with an appropriate reagent such as Woodward's reagent or EDC/NHS. The activated gel may then be reacted with, for example, proteins, antigen, or haptens. The gel can be formed with monomers having reactive functional groups of, for example, acrylic acid and acrylamide, e.g., from N-hydroxysuccinimidyl acrylate.

It has been demonstrated as disclosed herein that a gel described in the '470 patent is suitable for detecting biomolecular recognition events such as interactions between a captured antibody and an antigen. Unfortunately, the '470 patent described only a competitive immunoassay which uses a labeled molecule (see p. 4, col. 6, lines 52-65), such as a fluorescent tagged molecule to detect biomolecular interactions. This is a severe drawback in view of direct label-free methods that do not require labels, i.e., labeling the analyte of interest with fluorescent groups. Electrochemical detection is also described in the '470 patent as a potential use of the device including the described *in-situ* polymer gel.

The thick gels of the '470 patent may induce mass transport issues during the binding assay, that is, after protein capture the drug or small molecule must diffuse through the gel to reach the binding site of the protein. This diffusion limited process creates an obstacle for kinetic measurements and association/dissociation constant determination, such as Kₒₙ, K_{off}, and K_{d}. Another shortcoming of this chemistry occurs when it is used to prepare well-plates where polymer chains that grow into the thick gel layer are not anchored to the substrate and may be leached out during the assay and induce an unacceptable signal drift.

Despite of the high potential immobilization capacity available to the 3D gel structure, the '470 patent does not mention how to use such chemistry on, for example, a thin film LID resonant grating biosensor.

Other methods described in, for example, WO 2007/078873, entitled "Support for assaying analytes and methods of making and using thereof," and commonly owned and assigned U.S. Serial No. 61/123609, filed April 10, 2008, entitled "Surface for Label Independent Detection and Method Thereof," mention methods that are particularly compatible with LID biosensors and are relatively easy to implement. However, the methods may suffer from limited performance, which may be unacceptable for screening of drugs in HTS mode. That is, the activity of the protein and the amount of captured protein may be too low to provide a clear binding response. Brueggemeier et al. have described in Analytical Biochemistry, vol. 329, N°2, 2004, pages 180-189, the use of a separate crosslinker to form a hydrogel or a biosensor.

WO 2006/058237 discloses polymer-coated substrates for binding biomolecules and methods of making them, as well as their use.

In embodiments, the disclosure provides a label-free biosensor article comprising:
a substrate;
a tie-layer attached to the substrate surface; and
an acryloyl (i.e., acrylic-acrylamide) copolymer layer attached to the tie-layer;
the acrylic-acrylamide copolymer layer having a dry thickness, for example, of from 1 to 100 nanometers, from about 2 to about 75 nm, from about 2 to about 50 nm, from 3 to about 30 nm, from about 3 to about 20 nm, from about 5 to about 10 nm, including intermediate values and ranges, the acrylic-acrylamide copolymer can be prepared from, for example, a mixture of monomers of:
at least one acrylic acid or alkyl acrylic acid, and like monomers, or a combination thereof; and
at least one acrylamide, N-substituted acrylamide, N,N'-disubstituted acrylamide, alkyl acrylamide, N-substituted alkyl acrylamide, or N,N'-disubstituted alkyl acrylamide, and like monomers, or a combination thereof.

The substrate can be, for example, at least one of a plastic, a polymeric or a co-polymeric substance, a ceramic, a glass, a composite, a crystalline material, a metal, a noble metal, a semi-noble metal, a metallic oxide, a non-metallic oxide, a transition metal, or a combination thereof.

The acrylic-acrylamide copolymer can be prepared from, for example, a mixture of monomers of:
at least one acrylic acid or alkyl acrylic acid, and like monomers, or a combination thereof; and
at least one acrylamide, N-substituted acrylamide, N,N'-disubstituted acrylamide, alkyl acrylamide, N-substituted alkyl acrylamide, or N,N'-disubstituted alkyl acrylamide, and like monomers, or a combination thereof.

In a specific example, the acrylic-acrylamide copolymer can be prepared from a mixture of, for example, at least one of the acrylic acid monomers and at of the least one acrylamide monomers in a mole ratio of 1.0:2.0 to 2.0:1.0.

In embodiments, the acrylic-acrylamide copolymer can further include being cross-linked, the cross-linker being present, for example, in an amount of from 0.000 1 to 0.0015 mole percent based on the total moles of monomer, such as by normal addition or by superaddition.
The acrylic-acrylamide copolymer layer can further include, for example, at least one reactive group suitable for immobilizing a bioentity. An example of a suitable reactive group can be, for example, the reaction product resulting from treatment with NHS/EDC, such as an -NHS group, an s-NHS group, and like groups, or a combination thereof. The biosensor article can be activated with a suitable reagent to transform the cross-linked and substrate bound copolymer into a derivatized polymer surface having at least one reactive group such as NHS, and like reactive groups, which derivatized or activated surface permits or further enhances bioentity adsorption, immobilization, or both. The adsorbed or immobilized bioentity can have a molecular weight (M_{w}), for example, of up to about 60,000 Daltons, up to about 50,000 Daltons, up to about 40,000 Daltons, and up to about 30,000 Daltons, including intermediate values and ranges.

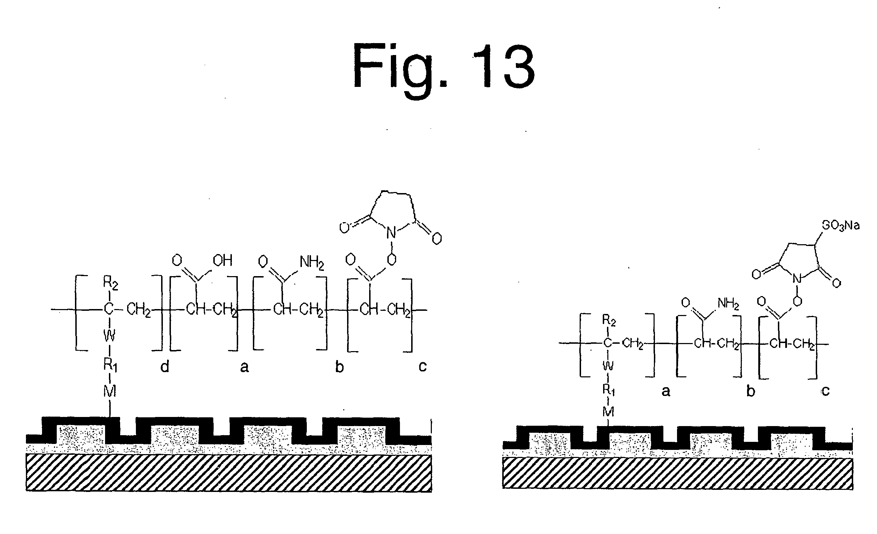

In embodiments, the disclosure provides a biosensor article comprising:
a substrate having a tie-layer attached to the substrate surface, and
a polymer layer attached to the tie-layer, the polymer being comprised of mers of the formula (I): 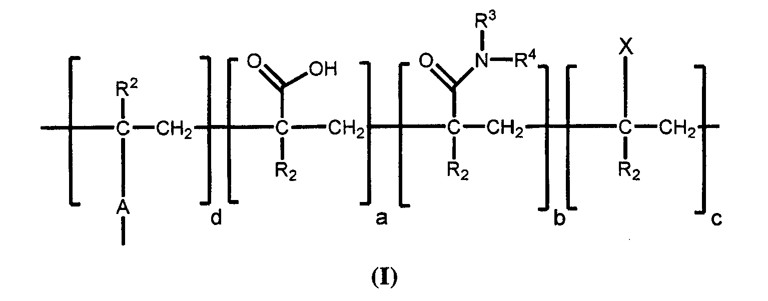
where:
***a*** comprises at least one acryloyl carboxylic acid group;
***b*** comprises at least one acryloyl amide group;
***c*** comprises at least one reactive group **X** suitable for immobilizing a bioentity, such as an NHS or s-NHS derived group as shown, for example, in the formulas of Fig. 13; and
***d*** comprises at least one tie-layer to surface attachment group A,
where
A is a tie-layer group attached to the substrate surface and co-polymerized with the acryloyl monomers;
**R²** is H or (C₁₋₄)alkyl; and
**R²** is H or (C₁₋₄)alkyl; and
**R³** and **R⁴** are each independently H or (C₁₋₄)alkyl,
the attached polymer layer having a dry thickness of, for example, from 5 to 50 nanometers, including all intermediate values and ranges. The polymer layer in aqueous media can have a swollen thickness of, for example, from about 10 to about 200 nanometers.

In embodiments, the polymer can be comprised, for example, of a mixture of acrylic acid and acrylamide monomers, in a mole ratio of 1.0:1.5 to 1.5:1.0. The polymer can further comprise an intrachain or interchain cross-linker (not shown) in an amount of from 0.001 to 0.0015 mole percent based on the total moles of monomer. In embodiments, a particularly effective cross-linker is N-N'-methylene bis-acrylamide, and like compounds.

The disclosure provides a method of making a biosensor as disclosed above, the method comprising:
contacting a substrate having a polymerizable tie-layer surface and a mixture of acrylic and acrylamide monomers and a cross-linking agent;
polymerizing the monomer mixture with the polymerizable tie-layer surface to form an initial polymer layer, the initial polymer layer having a thickness of, for example, from about 1 micron to about 100 microns; and
washing and drying the polymer layer to afford a residual polymer layer attached to the substrate having a thickness of from about 2 to about 100 nanometers, and the cross-linking agent being present at less than about 0.0015 mole % based on the total moles of monomer.

The washing of the polymer layer is believed to remove substantially all of the polymer that is unbound or unattached to the surface. The residual attached polymer layer can have a dry thickness, for example, of from about 5 to about 50 nanometers, and the cross-linking agent being present at less than about 0.0015 mole % based on the total moles of monomer. "Drying" and "dry" here refers to evaporation or removal of aqueous or non-aqueous liquids from the surface of the biosensor article, such as in air, in inert atmosphere, under reduced pressure, with mild heating, and like methods, or combinations thereof.

The method of making the biosensor article can further comprise an activating of the above resulting article by contacting the polymer with an activating agent.

The method of making the biosensor includes treating the substrate with a suitable tie-layer precursor compound to form a tie-layer modified surface. A tie-layer forming compound or tie-layer precursor compound can be, for example, of the formula:

**BₓM(C)₄₋ₓ,**

where
**M** is Si, Ti, Ta, Sn, Al, or a combination thereof,
**B** comprises a free-radical polymerizable group, each **B** is independently a substituted or unsubstituted, hydrocarbylene group having from 1 to about 18 carbon atoms,
**C** comprises a displaceable group, and
x is 1 to 3.

The **B** tie-layer surface group copolymerization with the acryloyl monomers can be accomplished, for example, by free-radical polymerization of the surface group(s) in the presence of the acryloyl monomers (i.e., the acrylic and acrylamide monomers). **B** surface groups can be for example, a free radical polymerizable group such as any of the following substituted or un-substituted groups that have or can be modified to have a carbon-carbon unsaturation or carbon-carbon double bond: alkyl, - alkoxy, -acryl, -alkyl acryl, -alkenyl, -cycloalkyl, -heterocyclic, -aryl, -heteroaryl, - alkylene-cycloalkyl, -cycloalkylene-alkyl, -alkylene-aryl, -alkylene-heteroaryl, - arylene-alkyl, -heteroarylen-alkyl, -alkylene-acryl, alkylene-alkyl acryl, and like hydrocarbyl groups, or mixtures thereof.

Each **C** displaceable group can be independently, for example, a halide, or a substituted or unsubstituted, hydrocarbyloxy group, also known as an alkoxy group, having from 1 to about 10 carbon atoms, which group can be partially or completely displaced from the M atom by, for example, one or more surface oxides.

The tie-layer precursor compound can be, for example, at least one compound of the formula:

(R¹)₂C = C(R¹)-Si(OR)₃,

{(R¹)₂C = C(R¹)-}₂Si(OR)₂,

{(R¹)₂C = C(R¹)-}₃Si(OR),

(R¹)₂C = C(R¹)-(CH₂)ₙ -Si(OR)₃,

{(R¹)₂C = C(R¹)-(CH₂)ₙ-}₂Si(OR)₂,

{(R¹)₂C = C(R¹)-(CH₂)ₙ -}₃Si(OR),

(R¹)₂C = C(R¹)-C(=O)- O-(CH₂)ₙ-Si(OR)₃,

{(R¹)₂C = C(R¹)-C(=O)-O-(CH₂)ₙ-}₂ Si(OR)₂,

{(R¹)₂C = C(R¹)-C(=O)-O-(CH₂)ₙ-}₃ Si(OR),

and like compounds, or a mixture thereof,
where n is an integer from 1 to about 16, each R is independently (C₁₋₁₀)alk and each R¹ is independently -H, or (C₁₋₁₀)alkyl.

The suitable tie-layer precursor compound can be, for example, of the formula {CH₂=C(CH₃)-C(=O)-O-(CH₂)₃-}Si(OR)₃ where R is methyl or ethyl. A excellent example of a tie-layer precursor compound or reactant can be, for example, methacryloxypropyltrimethoxysilane (MOPS).

The polymerizable acryloyl monomer mixture can be, for example, a combination of at least one acrylic acid or alkyl substituted acrylic acid monomer, and at least one acrylamide or alkyl substituted acrylamide monomer, and polymerizing the acryloyl monomer mixture can be accomplished by initiating polymerization using actinic radiation, a radiation sensitive initiator, or a combination thereof.

The disclosure provides a method of using the biosensor article a formula (I) described above, comprising contacting the activated polymer layer with a bioentity to absorb, immobilize, or a combination thereof, at least one bioentity.

In embodiments, the biosensor can be, for example, a resonance wave guide biosensor and such a biosensor can provide a bioentity immobilization response of, for example, about 1,500 pm or greater, such as measured with an Epic^{®} instrument.

In embodiments, the method of using the biosensor can further comprise, for example, contacting the absorbed or immobilized bioentity with a candidate ligand and detecting an interaction between the bioentity and the ligand. The detected interaction between the bioentity and the ligand using the biosensor can be, for example, from about 20 to about 200 pm. The method provides for at least about 20 wt% or more of the immobilized bioentity to bind with a ligand.

The disclosure provides a biosensor article or cell culture article prepared by the abovementioned preparative method.

In embodiments, a bioentity can be, for example, at least one of: a natural or synthetic oligonucleotide, a natural or synthetic nucleic acid (DNA or RNA), a natural peptide or synthetic peptide, a natural or synthetic peptide optionally comprising one or more modified or blocked amino acids, an antibody, a hapten, a biological ligand, a protein membrane, a lipid membrane, a protein, a small molecule having a molecular weight of less than about 1,000 Daltons, less than about 500 Daltons, and less than about 300 Daltons, a cell, and like bioentities, or a combination thereof, or a conjugate thereof.

In embodiments, the ligand can be, for example, at least one of: a stimulus, a therapeutic candidate, a prophylactic candidate, a prophylactic agent, a chemical compound, a biological molecule, a peptide, a protein, a biological sample, a small molecule having a molecular weight of less than about 1,000 Daltons, a biologic drug molecule candidate, a drug candidate small molecule-biologic conjugate, a pathogen, a cell, and like ligand entities, or combinations thereof.

In embodiments, the disclosure provides a label-free detection sensor, such as a resonant grating biosensor, comprising an *in-situ* formed synthetic polymer coated substrate for binding biomolecules and a method of making and using the coated sensor, such as for detecting or investigating ligand-bioentity interactions.

In embodiments, the disclosure provides a surface chemistry, based on acryloyl copolymers, which can be easily attached by, for example, *in situ* grafting to a properly (tie-layer) modified LID sensor surface, has very high bioentity immobilization capacity, providing good availability and activity of the immobilized biomolecule for ligand binding, and is compatible with label-free detection methodologies. The method of making is easy to implement, and is compatible with manufacture scale-up, e.g., does not require long surface polymerization times nor long washing times.

In embodiments, the present disclosure provides a polymer that is synthesized *in-situ* and directly on the LID sensor surface to overcome problems inherent in "grafting-to" methods. Up to now, commercially available LID biosensors have been prepared by grafting-to methods.

We have surprisingly discovered that a highly efficient LID sensor can be prepared using *in-situ* polymerization of, for example, methacrylic acid monomers, methacrylamide monomers, and like monomers, or a mixture thereof.

The LID sensor preparative process can include, for example:
grafting or attaching of a free radical co-polymerizable tie layer on the sensor surface; and
forming of an acrylic-based gel by *in-situ* free radical polymerization directly onto the LID sensor surface having the tie-layer and including a crosslinker in a concentration of from about 0.0001 to about 0.0015 mol% based on the monomer; and
eliminating any unanchored excess polymer formed *in-situ* by, for example, washing to remove the unbound excess polymer.

The free radical co-polymerizable tie layer can be made of, for example, an unsaturated condensed silane of the formula: **BₓM(C)₄₋ₓ,** as mentioned above, and like compounds, and combinations thereof.

The polymerization initiator preferably can be an electromagnetic source induced polymerization type, such as UV/Visible light and like actinic radiation initiated processes.

By using the described method, a very reproducible thin gel layer on the substrate can be obtained which is fully compatible with commercially available LID systems such as the Epic^{®} system (Coming Incorporated).

The thickness of the polymer layer was measured by, for example, an atomic force microscope (AFM). The AFM results indicated that the dry thickness of the attached polymer layer on the substrate was about 5 to about 50 nanometers and was considerably thinner than would have been expected. Although not limited by theory, the polymer layer in the form of a hydrated gel layer is believed to be thinner than previously reported acrylate gels. However, the density of the grafted acrylate polymer on the tie-layer modified substrate surface is believed to be significantly greater than for previously reported acrylate gels.

We have also discovered that when the gel is made of an acryloyl copolymer of acrylamide and acrylic acid monomers, or a substituted acrylic acid monomers (as described, for example, in "Polyacrylamide gels copolymerized with active esters. A new medium for affinity systems," R.L. Schnaar, et al., Biochemistry, Vol. 14, pgs. 1535-1541), the ratio of monomers is apparently significant to obtain high protein capture capacity and high protein activity. The monomer ratio can particularly significant in maintaining the biological activity of the attached protein which allows one to further take advantage of the large amount of protein captured. Conservation of a high activity ensures that a significant number of ligand analytes (the binding partner) will interact with the immobilized biologic target thus increasing the assay sensitivity and integrity.

In embodiments, the resonant grating biosensor can include, for example, an *in-situ* formed gel made of 50 mol% of methacrylic acid, and 50 mol% of an NHS-acrylate such as Sulfo-(N-hydroxy-succinimidyl(meth)acrylate).

In embodiments, the resonant grating biosensor can include, for example, an *in-situ* formed gel made of 50 mol% of an NHS- acrylate such as Sulfo-(N-hydroxysuccinimidyl(meth)acrylate) and 50 mol% of methacrylamide.

In embodiments, the resonant grating biosensor can include, for example, an *in-situ* formed gel made of 33 mol% methacrylic acid, 33 mol% NHS-acrylate such as Sulfo-(N-hydroxy-succinimidyl(meth)acrylate), and 33 mol% of methacrylamide.

Because a very high immobilization level of protein can be obtained and a high activity of the protein established, the coated sensor article of the disclosure can be particularly suitable for detecting binding events occurring between proteins and very low molecular weight molecules, e.g., small molecules, having a molecular weight, for example, of from about 75 to about 500 Daltons.

The LID sensor substrate, such as glass, plastic, and like substances, or combinations thereof, can be firstly coated or reacted with a tie-layer precursor before *in-situ* polymerization of the acrylic monomers. In embodiments, tie-layers can be comprised of, e.g., alkoxysilane or chlorosilane that can copolymerize or react with the acryloyl monomers to be polymerized. Such silanes include, for example, those bearing a vinyl group, a methacrylic acid ester group, an acrylate group, a (meth)acrylamide group, an acrylamide group, and like groups or combinations thereof. A preferred silane because of its availability, efficacy in copolymerizing, and efficiency of its copolymerized films in immobilization and ligand binding is, for example, a methacyloyloxypropyl trimethoxysilane of the formula:

CH₂ = CH- C(O)- O(CH₂)₃ - Si(OMe)₃.

The silane can be applied to the LID sensor surface, e.g., either by contact with a solution or a vapor phase. The *in situ* polymerization can be preferably performed using a solution of monomer, such as an organic or aqueous solution, or by plasma polymerization using a vapor of monomers. When acrylic acid is used in the monomer mixture to be polymerized *in situ,* the polymerization can be accomplished using an aqueous solution of monomers. An organic solvent solution may be preferred when a hydrolysis sensitive activated ester, such as N-Hydroxysuccinimidyl acrylate or its sulfonic acid derivative, is selected.

Alternative or additional reactive comonomers can be selected which comprise or can be converted after *in-situ* polymerization to aldehyde, carboxy, amide, azide, epoxy, isocyanate, isothiocyanate, maleimide, succinimidyl ester and succinmidyl carbonate, and like groups. A panel of reactive groups that can be used with the present disclosure is disclosed, e.g., in "Bioconjugate Techniques", Greg T. Hermanson, Academic Press, 1996. When the reactive group is likely to interfere with the in-situ polymerization of the monomers mixture, the reactive group can be, for example, introduced after the *in-situ* polymerization or can be protected using a protecting group.

Preferably, the monomer mixture to be polymerized *in-situ* contains from about 10% to about 90% of methacrylic monomer bearing a carboxylic acid group, or methacrylamide monomer, and more preferably from about 40% to about 60% of such monomers. Preferred acrylic monomers include acrylic acid and methacrylic acid. The total methacrylamide monomer concentration can be, for example, less than about 50 wt% and more preferably about 30 wt%. It is generally known that a higher monomer concentration will lead to a higher molecular weight of the polymer formed *in-situ.* A preferred reactive group is N-hydroxysuccinmidyl ester, and more preferred is sulfo-N-hydroxysuccinimidyl ester.

The present disclosure provides a process for preparing LID sensors having outstandingly high protein immobilization capacity while significantly improving the protein activity and dramatically enhancing the assay sensitivity. The immobilization and binding responses of the biosensor articles of the disclosure out-perform other known surface chemistries. For example, the Epic^{®} binding response for a furosemide/carbonic anhydrase assay as disclosed herein, is about 4 to about 5 times higher than the those obtained with a sensor made according to the method described in WO 2007/078873, and to our knowledge is the most effective immobilization and binding chemistry known for LID sensors.

In embodiments, the sensor article of the disclosure is compatible with high throughput screening (HTS) of drug or other small molecules due to the high binding response provided. Even very low molecular weight compounds, such as fragments having a molecular weight of less than about 500 Daltons, can be also screened using the sensor due to the high binding response.

The disclosure permits preparation of sensors for label-free detection using a low protein concentration which suggests that the cost per analysis can be substantially reduced compared to other LID techniques. The preparation of the sensor of the disclosure is straight forward; particularly, the washing step to remove unbound monomers and polymers, which requires, for example, only minutes instead of hours.

The disclosed method of making the surface modified biosensor provides for a significant reduction in the solvent used for manufacture compared with a typical dip coating process. For example, only 500 microliters of monomer solution can be used to prepare a 384-well plate. The manufacture process can also be very efficient because the surface chemistry can be quickly and easily affixed to the sensor plate, for example within a few seconds, using UV irradiation.

Unlike prior art techniques, the sensor and sensing method of the disclosure allow kinetic measurement of association/dissociation between a ligand and an analyte because of the thin gel layer used. The sensor plate made according to the disclosure exhibits very good response uniformity across the entire area of the plate, which is particularly significant in a HTS mode which requires a highly reliable response from all wells.

In embodiments, the disclosure provides a surface that is suitable for the attachment, growth, and assay of many types of cells, including strongly adherent cells such as Chinese hamster ovary (CHO) cells and human epithelial carcinoma A431 cells, intermediate adherent cells such as RMS 13 cells, and weakly adherent cells such as human embryonic kidney (HEK) cells, or primary cells.

The disclosure provides methods to modify the surface of a biosensor so that the surface of these biosensors is compatible with and amenable to cell culture and subsequent cell assays. The disclosed method is suitable for oxidized metal thin film surfaces such as the ones used in resonant waveguide grating biosensors, or an unpatterned gold surface, such as those used in surface plasmon resonance (SPR), or a patterned gold surface, such as those used in electrical bioimpedance-based biosensors.

Thus, the disclosure may suitably comprise, consist of, or consist essentially of: a cell culture article as defined herein; a method for preparing the cell culture article as defined herein; and a method for performing an assay of a ligand as defined herein.

The disclosure provides a cell culture article comprising:
a substrate;
a tie-layer attached to at least the substrate; and
a bio-compatible layer attached to the tie layer, to the substrate, or both.

The substrate can comprise, for example, a plastic, a polymeric or co-polymeric substance, a ceramic, a glass, a metal, a crystalline material, a noble or semi-noble metal, a metallic or non-metallic oxide, a transition metal, or a combination thereof. In embodiments, the tie-layer can be obtained from a compound comprising one or more reactive functional groups comprising, for example, an amino group, a thiol group, a hydroxyl group, a carboxyl group, an acrylic acid, an organic or inorganic acid, an ester, an anhydride, an aldehyde, an epoxide, and like groups, and salts thereof, or a combination thereof. The choice of materials for forming the tie-layer can depend on the nature of the substrate. For example, silane can be an excellent tie-layer in conjunction with an oxidized inorganic substrate such as glass, SiOₓ-presenting substrate, TiO₂, Nb₂O₅, Ta₂O₅, HfO₂, and mixtures thereof, or like substrate. Alternatively or additionally, the aforementioned inorganic substrates can be combined with a SiOₓ overlay. A thiol compound can be an excellent tie-layer when a gold substrate is selected. A positively charged polymer such as poly-lysine can be an excellent tie-layer when a polymeric substrate is used.

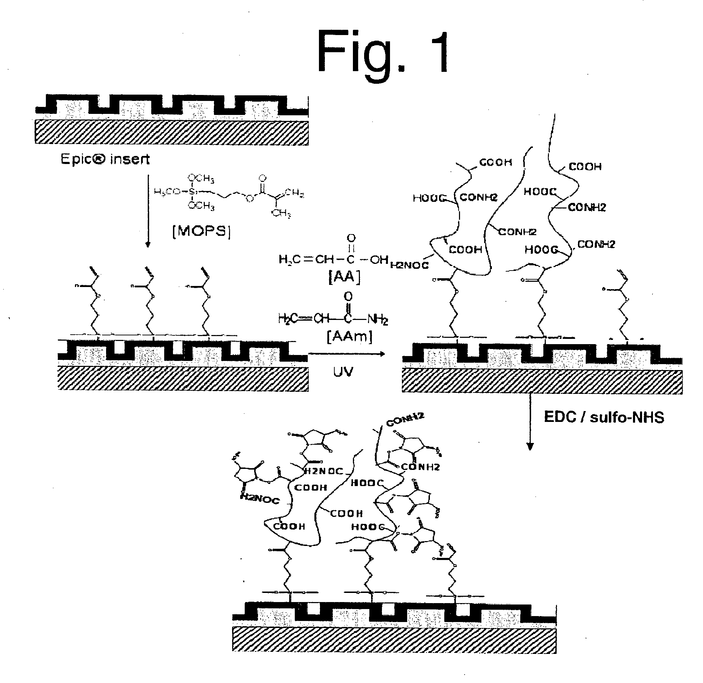

Referring to the Figures, Fig. 1 is a schematic of the steps used to form the polymer *in situ* on the LID biosensor surface, including: i) MOPS silane treatment; ii) *in situ* polymerization of the mixture of monomers with UV irradiation; and iii) activation of the polymer gel by EDC/Sulfo-NHS treatment.

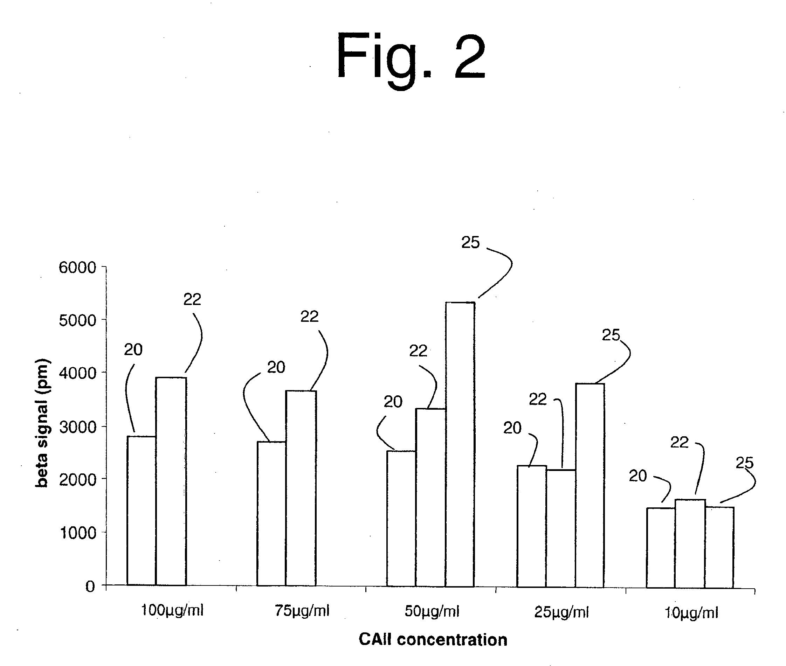

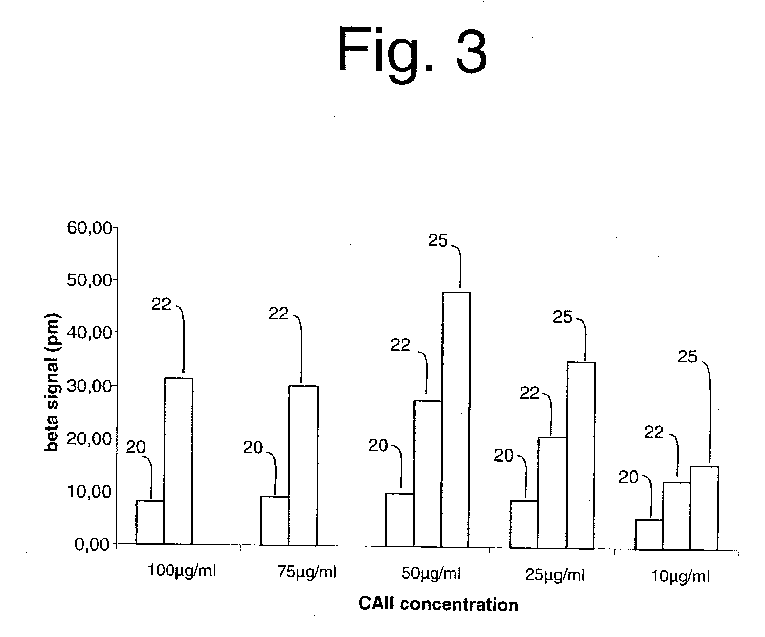

Figs. 2 and 3 show graphs of carbonic anhydrase II (CAII) immobilization response in acetate buffer measured with an Epic^{®} instrument on biosensors having the inventive (25) and the comparative surface (20, 22) chemistries. The inventive example of the disclosure is a 50/50 or 1:1 (mol:mol) acrylic acid and acrylamide copolymer (25), comparative example 2 is the "spacer" chemistry (22), and comparative example 1 is the "dEMA" chemistry (20). Assuming a sensitivity factor of about 4.5 pg/mm²/pm, the density of bound CAII is about 24 ng/mm². The maximum capacity of the surface chemistry is higher than this value because the concentration of protein used limits the immobilization response (i.e., a plateau not reached).

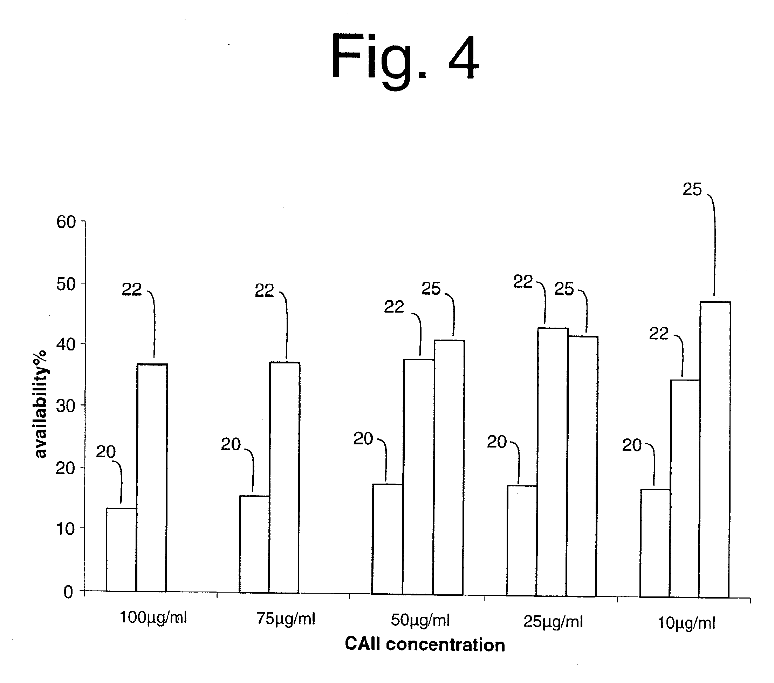

Fig. 4 shows a graph of carbonic anhydrase (II) availability (%) as a function of concentration for the disclosed and comparative surface chemistries. This figure shows the extraordinary availability%, even at CAII concentration of 10 micrograms/mL.

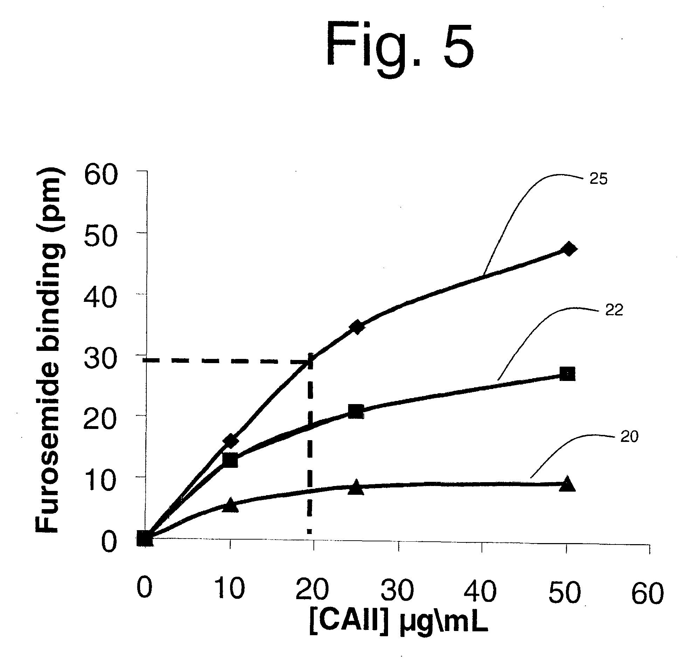

Fig. 5 shows a graph of furosemide drug binding response versus CAII concentration used for the immobilization on the inventive and the comparative prepared surfaces. The example of the disclosure was a 50/50 or 1:1 (mol:mol) acrylic acid and acrylamide copolymer (25), comparative example 2 was the "spacer" surface (22), and comparative example 1 was the "dEMA" surface (20). This figure shows that the protein consumption can be significantly reduced for a given binding response using the disclosed prepared surface (**25**). For example, a 30 pm response can be obtained using 20 micrograms/milliliter of protein with the inventive biosensor surface. In contrast, more than 50 micrograms/milliliter are required with the biosensor surface of comparative example 2 (**22**), and cannot be reached with the low capacity biosensor surface of comparative example 1 (**20**).

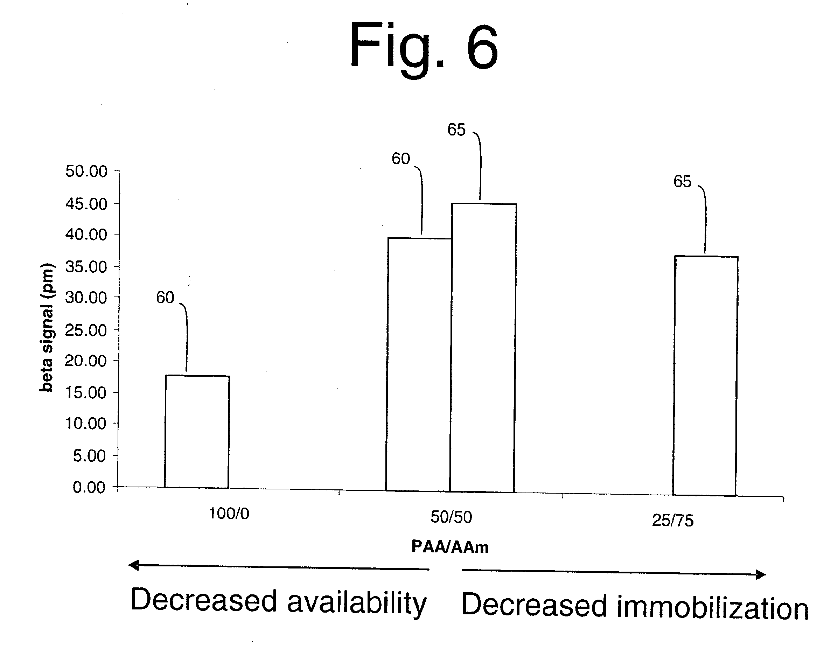

Fig. 6 shows a graph of the influence of for the disclosed surfaces on the furosemide binding response to CAII immobilized at 50 microgram/mL at 4°C in acetate buffer (20 mM; pH 5.5), measured on a first date (**60**) and a second later date (**65**). The acrylic acid/acrylamide monomer mole ratios in the copolymer surface compositions shown are 100/0, 50/50, and 25/75, respectively. The graph shows that an optimum monomer mole ratio for superior binding was, for example, at about 50/50. A large ratio of acrylic acid (25 parts) to acrylamide (75 parts) content increased the protein activity but reduced protein capture, i.e., decreased CAII immobilization, leading to a decrease of the binding response. On the contrary, a large or exclusive acrylic acid content (100/0) increased the protein immobilization but decreased the protein activity, i.e., decreased availability, which also lead to a decreased binding response.

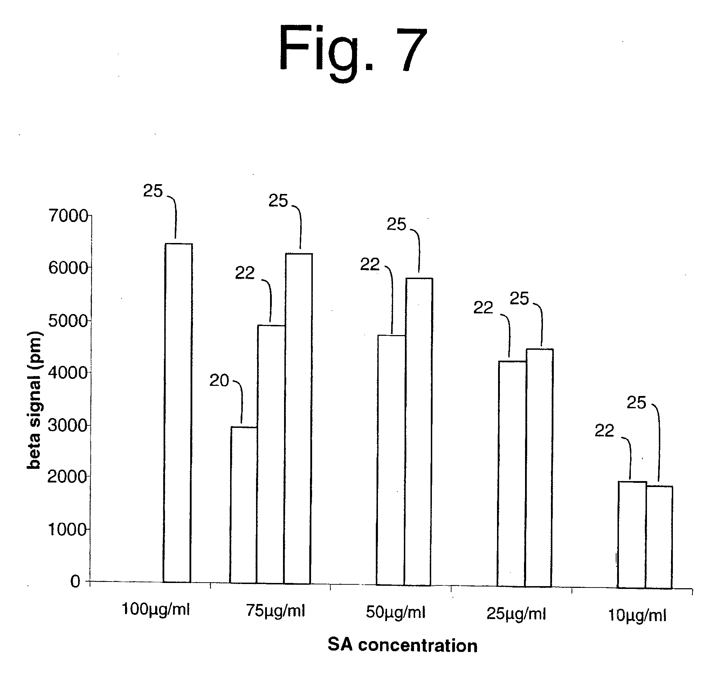

Fig. 7 shows a graph of streptavidine (SA) immobilization response for the disclosed and the comparative prepared surfaces measured with an Epic^{®} instrument. The SA was immobilized in acetate buffer (20 mM; pH 5.1). The example of the disclosure was a 50/50 or 1:1 (mol:mol) acrylic acid and acrylamide copolymer (**25**), comparative example 2 was the "spacer" surface (**22**), and comparative example 1 was the "dEMA" surface (**20**). Consistently higher immobilization over the comparative examples was demonstrated except for comparable immobilization observed at the lowest SA concentration level (10 micrograms/mL).

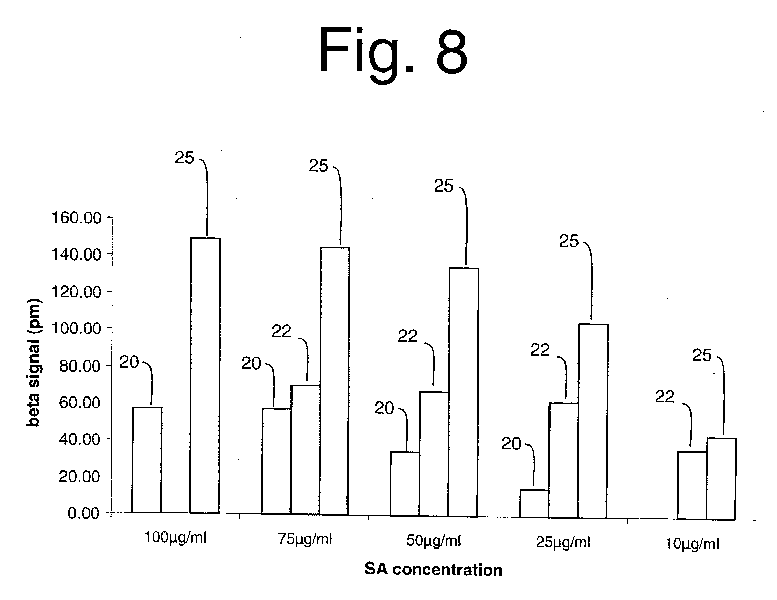

Fig. 8 shows a graph of biotin binding response for the inventive and the comparative prepared surfaces measured with an Epic^{®} instrument. The biotin binding was measured against immobilized SA in acetate buffer (20 mM; pH 5.1). The inventive example of the disclosure was a 50/50 or 1:1 (mol:mol) acrylic acid and acrylamide copolymer (**25**), comparative example 2 was the "spacer" surface (**22**), and comparative example 1 was the "dEMA" surface (**20**). A consistently large binding response differential for the inventive example compared with comparative examples 1 and 2 was observed at each the SA concentrations tested. Assuming a sensitivity factor of about 4.5 pg/mm²/pm, the density of bound CAII was about 30 ng/mm².

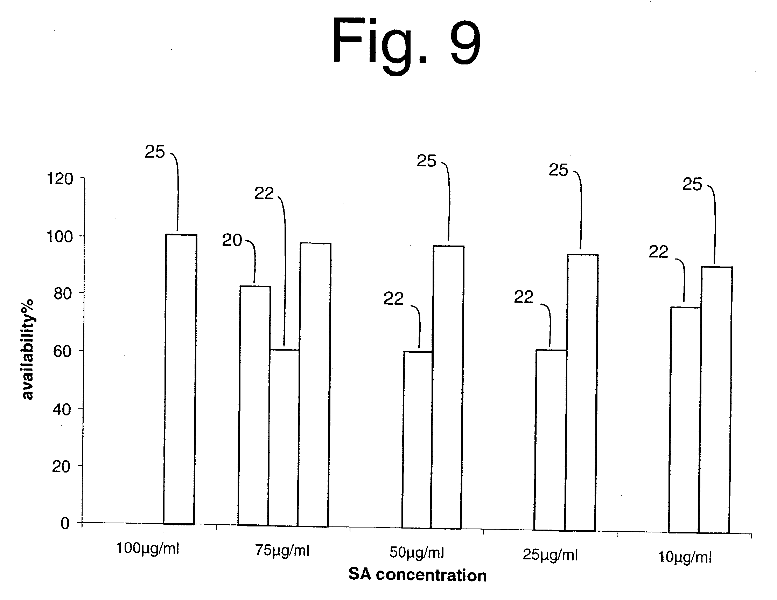

Fig. 9 shows a graph of calculated availability of the immobilized SA in view of availability, expected binding response, relative Refractive Increase Increment (RII), and molecular weight of the receptor and analyte. The expected binding response assumes an analyte:receptor ratio of 4:1 (stoichiometry). The disclosed example of the disclosure was a 50/50 or 1:1 (mol:mol) acrylic acid and acrylamide copolymer (**25**), comparative example 2 was the "spacer" surface (**22**), and comparative example 1 was the "dEMA" surface (**20**). This graph demonstrates that the SA activity was about 100% which show that the full biological activity of the protein has been maintained upon its attachment to the surface.

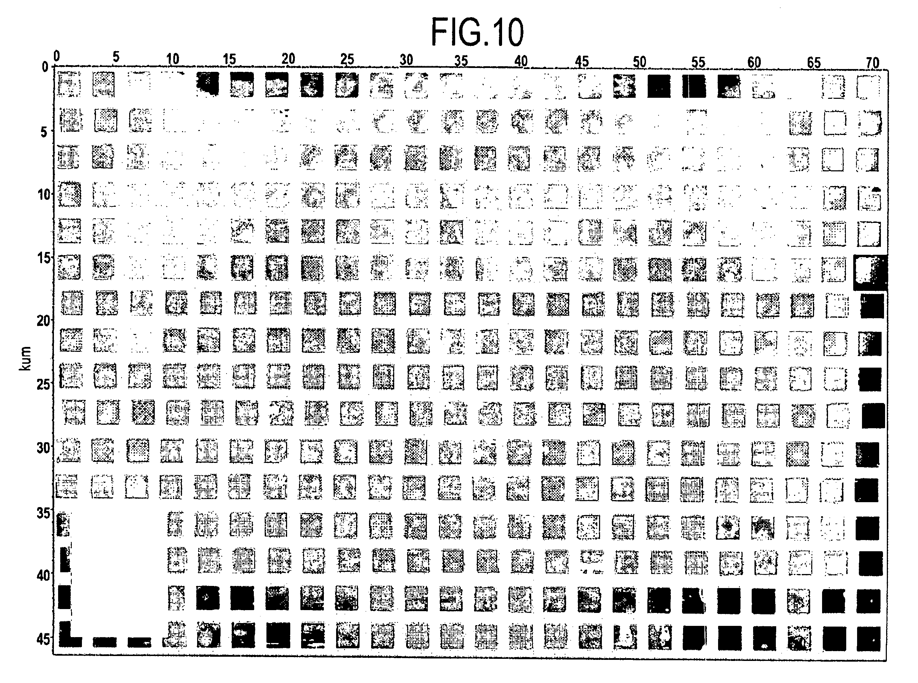

Fig. 10 shows a photograph illustrative of the non-uniformity of CAII immobilization in a 384-well plate having a comparative surface using the Epic^{®} 2D Map software. Observed green and blue grating areas (color not shown; i.e., the light gray squares in gray scale), correspond to those well areas where proteins have been immobilized and wells where only buffer was added, respectively. The well areas where proteins have been immobilized cannot be easily distinguished from well areas having only buffer. This plate exhibits poor immobilization uniformity and has high variability.

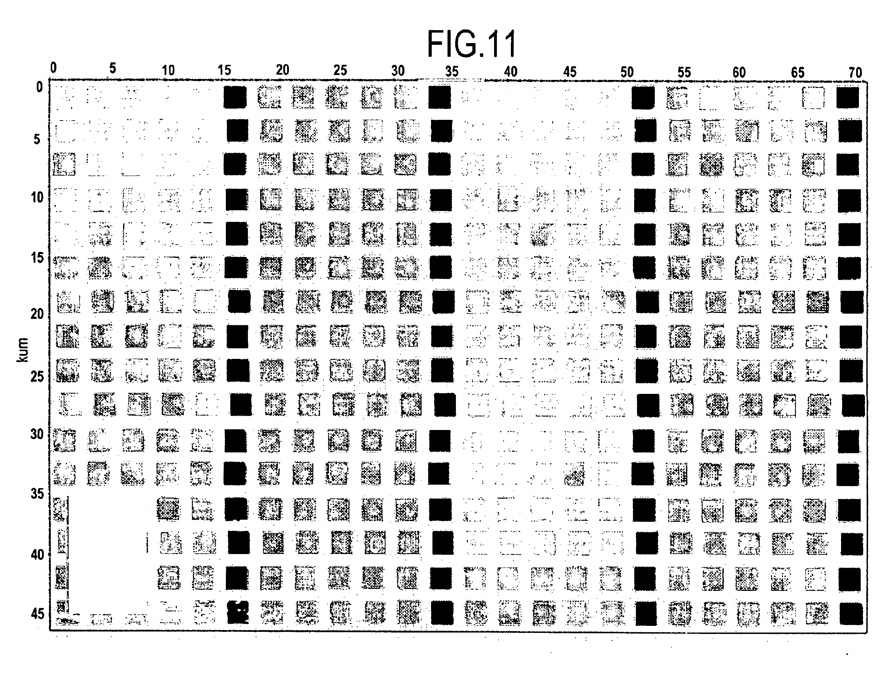

Fig. 11 shows a photograph illustrative of the uniformity of CAII immobilization in a 384-well plate having an inventive surface using the Epic^{®} 2D Map software. Green and blue grating areas (respective colored regions are not shown nor apparent in gray scale) correspond to well areas where proteins have been immobilized (columns 1 to 5, 7 to 11, 13 to 17, and 19 to 23) and wells where only buffer was added (i.e., dark gray columns) (columns 6, 12, 18, 24), respectively. The plate exhibited excellent immobilization uniformity, that is, for example, less than about 0.5 pm variability.

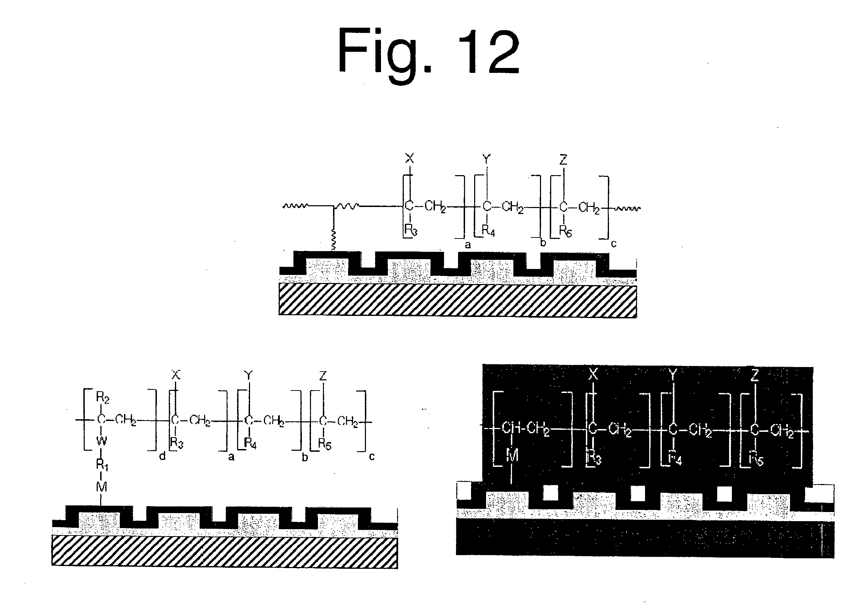

Fig. 12 is a schematic of an illustrative sensor and polymer layer structure combinations. Examples of schematic structures of the of the disclosure are shown based on the formula (I). The *in-situ* formed polymer gel is attached to the sensor surface or to the tie-layer modified sensor by at least one covalent bond or group, such as -A- in formula (**I**) above, or alternatively as -W-R₁-M-,
where, for example:
M is a surface metal or a metal oxide, for example, Si or Ti oxides attached to the sensor substrate by at least one covalent bond;
R¹ is, for example, an alkyl or aromatic or like divalent linker, such as propyl or phenyl;
R², R³, and R⁴ can be, for example, independently H or CH₃;
W can comprise, for example, a divalent linker group, such as amide (-C(=O)-NR-) or preferably a carboxy or ester group (-C(=O)-O-), and like groups;
X can comprise, for example, a carboxylic acid (-CO₂H), sulfonic acid (-SO₃H), or phosphoric (-O-P(O)(OH)₂) or phosphonic acid (-P(O)(OH)₂), and like groups, more preferably a carboxylic acid;
Y can comprise, for example, an amino (-C(=O)-NR₂) group or substituted amide group, and like groups;
Z can comprise, for example, a group that can be converted into, that is activated to bear or have a reactive group as illustrated and demonstrated herein such as an NHS or sulfo-NHS group; and
a, b, c, and d can be, for example, integers from 1 to about 100.

In embodiments, (***a*** + ***c***) can be greater than or equal to ***b***, and, for example, **(*a* + *c*)** ≥ ***b*** >> ***d**.* If, for example, **(*a* + *c*):*b*** = 1:1 then ***d*** can be, for example, from about 0.001 to about 0.2, from about 0.001 to about 0.1, or from about 0.001 to about 0.01.

Fig. 13 is a schematic of illustrative exemplary activated polymer structures which include, for example, those having reactive groups appended to the "c" mer, such as an NHS or sulfo-NHS group.

In embodiments, the copolymer layer can have a dried thickness, that is after washing, for example, of from about from about 1 to about 200 nm, from about 2 to about 150 nm, from about 2 to about 100 nm, and from about 2 to about 50 nm, including all intermediate values and ranges.

The surface chemistry enhancements of the present disclosure are applicable to other substrates including other glasses, metals, plastic substrates, such as Topas^{®} COC substrates, available from TOPAS Advanced Polymers, Inc., and like materials, or a combination thereof. Commonly owned and assigned copending U.S. Patent Application 12/201,029, filed August 29, 2008, mentions plasma treated cyclic polyolefin copolymer surfaces having enhanced binding density for biologically active agents and cells. These plasma treated cyclic polyolefin copolymer surfaces may be further enhanced for binding biologically active agents or cells using conjugates.

In embodiments, the interacting ligand can comprise, for example, a complementary entity to the bio-material associated with the bio-compatible layer, such as a stimulus, a therapeutic compound, a therapeutic candidate, a prophylactic candidate, a prophylactic agent, a chemical compound, a biological molecule, a peptide, a protein, a biological sample, a drug candidate small molecule having a molecular weight of, for example, less than about 500 Daltons, a drug candidate biologic molecule, a drug candidate small molecule-biologic conjugate, a pathogen or combinations thereof. In embodiments, the bound ligand can be detected by any suitable method, for example, fluorescence, label-independent-detection methods, including optical biosensors such as a waveguide resonant grating (RWG) system, surface plasmon resonance (SPR), impedance, mass spectrometry, and like methods.

### EXAMPLES

The following examples serve to more fully describe the manner of using the above-described disclosure, as well as to set forth the best modes contemplated for carrying out various aspects of the disclosure. It is understood that these examples in no way limit the scope of this disclosure, but rather are presented for illustrative purposes. The working examples describe how to prepare and use the LID sensor of the disclosure which are contrasted with the comparative examples.

### EXAMPLE 1

A 2 wt % methacryloxypropyltrimethoxysilane (MOPS) (from Sigma Aldrich) aqueous solution containing acetic acid to give a pH of about 3.5 to about 4 was prepared and stirred until clear (typically 15 min at about 22°C).

A bare Epic^{®} glass insert used as received without additional cleaning step was contacted with the MOPS solution for 2 hours then dried under argon and air dried at 50°C for 1 hour to ensure condensation of the silane with the insert glass surface. Then the insert was cooled to RT, rinsed with de-ionized (DI) water then with isopropyl alcohol and finally dried with argon.

The MOPS treated insert was contacted with approx. 5-10 micron-thick layer of an aqueous monomer mixture of acrylamide (50.3 mole %), acrylic acid (49.6 mole %) and Irgacure 184 mole %. 20 mg of Irgacure 184™ (from Ciba) were weighted into a flask and 7 ml of DI water added. Then 1.5 g of acrylic acid and 1.5 g of acrylamide was added. The flask was sealed with a septum and the solution was degassed under bubbling argon for 15 min at 500 microliters of the degassed monomer mixture was deposited onto the silane treated insert surface. The thickness of the monomer aqueous solution was achieved by clamping a hydrophobic treated glass plate (FDS fluorosilane). *In-situ* polymerization was performed under UV light using a fusion lamp (bulb H, 80% power, 5 passes at the minimum belt speed). Then the cover plate was removed and the adhering gel layer was thoroughly rinsed with DI water in an ultrasonic bath to remove excess of materials and the surface was finally dried under a gentle argon flow. Following polymerization and cleaning, the Epic^{®} insert was assembled with a Coming Inc. holey-plate using PSA adhesive tape.

The carboxylic acid groups contained within the polymer matrix were activated (using a Biomek automation workstation) by treatment with 50 mM sulfo-NHS and 200 mM EDC dissolved in water. At this step, the activated copolymer may be reacted with protein, or another biomolecule that can be immobilized by amine coupling. This plate was referred to as "PAA/AAm 50/50".

### COMPARATIVE EXAMPLE 1

An Epic^{®} insert was functionalized according to Example 1 and 9 of WO 2007/078873 (US Application No. 11/448,486). The surface chemistry used for this example is based on ethylene-maleic anhydride copolymer partly derivatized by propylamine. The protein was captured by amine coupling by reaction between amino groups of the protein and anhydride groups for the attached polymer. This plate was referred to as "dEMA".

### COMPARATIVE EXAMPLE 2

An Epic^{®} insert was functionalized according to Example I of commonly owned and assigned copending patent application, U.S. Serial No. 61/123609, filed April 10, 2008, entitled "Surface for label independent detection and method thereof." This surface chemistry involves an ethylene-maleic anhydride copolymer attached to the sensor surface. The copolymer being partly derivatized with a carboxyl PEG spacer and propylamine, and activated for amine coupling by sulfo N-hydroxysuccinimide (Sulfo-NHS). A commercial Epic^{®} Plate (ID 38043) was treated as follow: 15 microliters of 10 mM AminoPEG-4acid spacer (from QuantaBiodesign) in borate buffer pH 9.2 was added to each well. The solutions were mixed using the automated pipettor and the plate is spun down for 1 min to eliminate trapped bubbles and then incubated 30 min at room temperature. After incubation, the plate was rinsed three times with water. After rinsing, 10 microliters of 200 mM EDC/50 mM sulfo-NHS in DMSO or water was added in each well via 16-channel hand pipettor. The plate was spun down for 1 min at 800 rpm. The plate was left undisturbed for 30 min at room temperature. Finally, the plate was washed with a plate washer. This plate was referred to as "spacer" surface.

### EXAMPLE 2

**Carbonic anhydrase II (CAII) immobilization and CAII/Furosemide binding assay** CAII immobilization was performed at 50, 25 and 10 g/mL protein in 20 mM acetate buffer pH 5.5 on the LID sensor of the disclosure and at 100, 75, 50, 25 and 10 g/mL protein in 20 mM acetate buffer pH 5.5 on a sensor surface prepared according to the comparative examples. The plate was incubated overnight at 4°C to complete immobilization.

After equilibration a RT, the plate was rinsed with PBS (using an automated workstation) and 15 microliters of PBS containing 0.1% DMSO in each well. Then furosemide was added to reach a final concentration of 10 µM.

Both immobilization response and binding response were recorded using the Epic^{®} instrument. Immobilization response and binding responses are provided in Fig. 4 and Fig. 5, respectively.

### EXAMPLE 3

**Immobilization of Streptavidine (SA) and SA/biotin binding assay SA** immobilization was performed at 100, 75, 50, 25 and 10 micrograms/mL protein in 20 mM acetate buffer pH 5.1 on the LID sensor of the disclosure and comparative examples. The plate was incubated overnight at 4°C to complete immobilization. After equilibration a RT, the plate was rinsed with PBS (using the Biomek automation workstation) and 15 microliters of PBS containing 0.1% DMSO in each well. Then biotin was added to reach a final concentration of 5 µM. Both immobilization response and binding response were recorded using the Epic^{®} instrument. Exemplary immobilization responses are given in Fig. 7, 8, and 9.

### COMPARATIVE EXAMPLE 3

This example describes the preparation of a LID sensor according to the '470 patent examples. 2 wt % methacryloxypropyltrimethoxysilane (MOPS) (from Sigma Aldrich) aqueous solution containing acetic acid to give a pH of 3.5-4 was prepared and stirred until clear (typically 15 min at about 22°C). A bare Epic^{®} insert, used as received an without additional cleaning step, was contacted with the solution for 2 hours then dried under argon flow and dried at 50°C for 1 hour to ensure condensation of the silane with the insert surface. Then the insert was cooled to RT, rinsed with DI water then with isopropyl alcohol (IPA) and finally dried with an argon stream.

The MOPS treated insert was then treated as in Example 1 above with the exception that 1 mol% of N-N'-methylene bisacrylamide cross-linker was added and there was no rinsing step of the resulting adherent gel layer as described in Example 5 of the '470 patent.

Briefly, a viscous aqueous monomer solution layer on a substrate surface was clamped together with a hydrophobic treated glass cover plate (FDS fluorosilane). In-situ polymerization was performed under UV light using a fusion lamp (bulb H, 80% power, 5 passes at the minimum belt speed). Then the cover plate was removed and the Epic^{®} insert was assembled with a Corning^{®} holey-plate using pressure sensitive adhesive (PSA) tape. The thickness of the final gel obtained was about 10 microns.

The carboxylic acid groups contained within the polymer matrix were activated (using a Biomek automation workstation) by treatment with 50 mM sulfo-NHS and 200 mM EDC dissolved in water.

### COMPARATIVE EXAMPLE 4

**Carbonic anhydrase II (CAII) immobilization and CAII/Furosemide binding assay.** CAII immobilization was performed at 100 µg/ml protein in 20 mM acetate buffer pH 5.5. No protein was added in columns 6, 12, 18, and 24, but rather only acetate buffer. The plate was incubated overnight at 4°C to complete immobilization. After equilibration at RT, the plate was rinsed with PBS (using an automated workstation) and 15 microliters of PBS containing 0.1% DMSO added in each well. Then furosemide was added to reach a final concentration of 10 micromolar.

The immobilization response and the binding responses were recorded using the Epic^{®} instrument. Total immobilization response and binding signal are provided in the accompanying Tables 1 and 2, respectively.

**Table 1. Total immobilization response of Comparative Example 4.**

| **Group No.** | **Positive Wells (x)** | **Positive Wells (s)** | **Negative Wells (x)** | **Negative Wells (s)** | **Total Immob.** |
|---|---|---|---|---|---|
| 1 | 832463.77 | 319.16 | 832398.80 | 383.47 | 64.97 |

**Table 2. Total binding signal of Comparative Example 4.**

| **Group No.** | **Pos Wells (x)** | **Pos Wells(s)** | **Neg Wells (x)** | **Neg Wells (s)** | **Total Signal (x)** | **CV %** | **Z'** |
|---|---|---|---|---|---|---|---|
| **1** | **-437.17** | **132.99** | **-418.97** | **88.99** | **-18.20** | **-879.16** | **37.59** |

The total immobilization of CAII was less than about 65 pm compared to about 6,100 pm with the sensor prepared according to the present disclosure. This result demonstrates that immobilization(s) cannot be detected using a thick gel prescribed by the preparative procedures of the '470 patent.

A 2D MAP analysis of the comparative plate, shown in Fig. 10, indicated no discernable difference between the columns (i.e., wells) having immobilized proteins and columns having only buffer added, i.e., without proteins. In contrast, as shown in Fig. 11, buffer only columns, i.e., dark shaded vertical columns, were clearly distinguishable from wells having immobilized protein using the inventive sensor surface of the disclosure.

Additionally, the furosemide binding response was not detectable using the plate prepared and tested in accord with comparative example 4 because of the negative values obtained (-18.2 pm) compared to 44 pm with sensor of the present disclosure.

The absence of evidence for an immobilization and binding response demonstrated that the thick gels set forth in the examples of the '470 patent are inoperable and therefore unsuitable for LID detection sensors.

## Claims

1. A label-free biosensor article comprising:
a substrate;
a tie-layer attached to the substrate surface; and
an acrylic-acrylamide copolymer layer attached to the tie-layer;
wherein the acrylic-acrylamide copolymer layer having a dry thickness of from 1 to 100 nanometers, and
wherein the acrylic-acrylamide copolymer comprises a mixture of monomers of:
at least one acrylic acid or alkyl acrylic acid, or a combination thereof; and
at least one acrylamide, N-substituted acrylamide, N,N'-disubstituted acrylamide, alkyl acrylamide, N-substituted alkyl acrylamide, or N,N'-disubstituted alkyl acrylamide, or a combination thereof.

2. The article of claim 1 wherein the substrate comprises at least one of a plastic, a polymeric or a co-polymeric substance, a ceramic, a glass, a composite, a crystalline material, a metal, a noble metal, a semi-noble metal, a metallic oxide, a non-metallic oxide, a transition metal, or a combination thereof.

3. The article of claim 1 wherein the acrylic-acrylamide copolymer comprises a mixture of at least one of the acrylic acid monomers and at of the least one acrylamide monomers in a mole ratio of 1.0:2.0 to 2.0:1.0.

4. The article of claim 1 further comprising the acrylic-acrylamide copolymer being cross-linked, the cross-linker being present in an amount of from 0.0001 to 0.0015 mole percent based on the total moles of monomer.

5. The article of claim 1 further comprising the acrylic-acrylamide copolymer having at least one reactive group suitable for immobilizing a bioentity.

6. A biosensor article comprising:
a substrate having a tie-layer attached to the substrate surface, and
a polymer layer attached to the tie-layer, the polymer being comprised of mers of the formula (I): 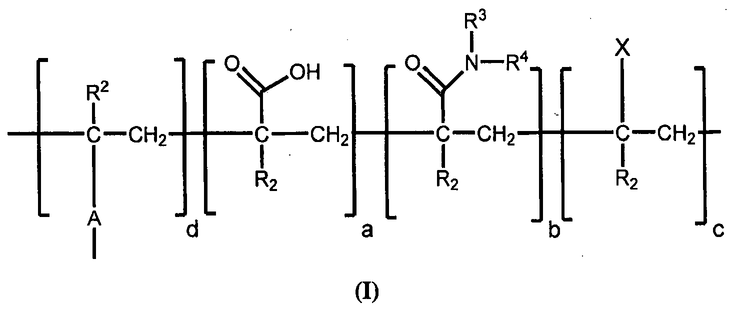 where:
a comprises at least one acryloyl carboxylic acid group;
b comprises at least one acryloyl amide group;
c comprises at least one reactive group X suitable for immobilizing a bioentity; and
d comprises at least one tie-layer to surface attachment group A,
where
**A** is a tie-layer group attached to the substrate surface and co-polymerized with the acryloyl monomers;
**R²** is H or (C₁₋₄)alkyl; and
**R³** and **R⁴** are each independently H or (C₁₋₄)alkyl,
the polymer layer having a dry thickness of from 5 to a 50 nanometers.

7. The article of claim 6 wherein the polymer comprises a mixture of acrylic acid and acrylamide monomers in a mole ratio of 1.0:1.5 to 1.5:1.0, and the polymer further comprises a cross-linker in an amount of from 0.001 to 0.0015 mole percent based on the total moles of monomer.

8. The article of claim 6 wherein the polymer layer in aqueous media has a thickness of from 50 to 300 nanometers.

9. A method of using the biosensor article of claim 5 comprising contacting the activated polymer layer with a bioentity to absorb, immobilize, or a combination thereof, at least one bioentity.

10. The method of claim 9 wherein the biosensor is a resonance wave guide biosensor and provides a bioentity immobilization response of 1,500 pm or greater.

11. The method of claim 9 further comprising contacting the absorbed or immobilized bioentity with a candidate ligand and detecting an interaction between the bioentity and the ligand.

12. The method of claim 11 wherein the detected interaction between the bioentity and the ligand with biosensor is from 20 to 200 pm.

13. The method of claim 11 wherein 20 wt% or more of the immobilized bioentity binds with a ligand.

14. The article of claim 6 wherein the biosensor is a resonant wave guide type.

15. The article of claim 5 wherein the reactive group is a sulfo-NHS ester.

## Patentansprüche

1. Markerfreier Biosensorgegenstand, umfassend:
ein Substrat,
einen an der Substratoberfläche anhaftenden Haftvermittler und
eine an dem Haftvermittler anhaftende Acryl-Acrylamid-Copolymerschicht,
wobei die Acryl-Acrylamid-Copolymerschicht eine Trockendicke von 1 bis 100 Nanometer aufweist und
wobei das Acryl-Acrylamid-Copolymer eine Mischung von Monomeren umfasst aud:
wenigstens einer Acrylsäure oder einer Alkylacrylsäure oder einer Kombination davon und
wenigstens einem Acrylamid, einem N-substituierten Acrylamid, einem N,N'-disubstituierten Acrylamid, einem Alkyl-Acrylamid, einem N-substituierten Alkyl-Acrylamid oder einem N,N'-disubstituierten Alkyl-Acrylamid oder einer Kombination davon.

2. Gegenstand nach Anspruch 1, wobei das Substrat wenigstens eines von einem Kunststoff, einer Polymer- oder einer Copolymer-Substanz, einem Keramik, einem Glas, einem Verbundstoff, einem Kristallmaterial, einem Metall, einem Edelmetall, einem Halbedelmetall, einem Metalloxid, einem Nichtmetalloxid, einem Übergangsmetall oder einer Kombination davon umfasst.

3. Gegenstand nach Anspruch 1, wobei das Acryl-Acrylamid-Copolymer eine Mischung von wenigstens einem der Acrylsäuremonomeren und wenigstens einem der Acrylamidmonomere in einem Molverhältnis von 1,0:2,0 bis 2,0:1,0 umfasst.

4. Gegenstand nach Anspruch 1, ferner umfassend, dass das Acryl-Acrylamid-Copolymer quervernetzt ist, wobei der Quervernetzer in einem Gehalt von 0,0001 bis 0,0015 Molprozent basierend auf der Gesamtmolzahl der Monomere vorhanden ist.

5. Gegenstand nach Anspruch 1, ferner umfassend, dass das Acryl-Acrylamid-Copolymer wenigstens eine reaktive Gruppe aufweist, die zur Immobilisierung eines Bioobjektes geeignet ist.

6. Biosensorgegenstand, umfassend:
ein Substrat mit einem an der Substratoberfläche anhaftenden Haftvermittler und
einer an dem Haftvermittler anhaftenden Polymerschicht, wobei das Polymer aus Monomeren der Formel (I) besteht: 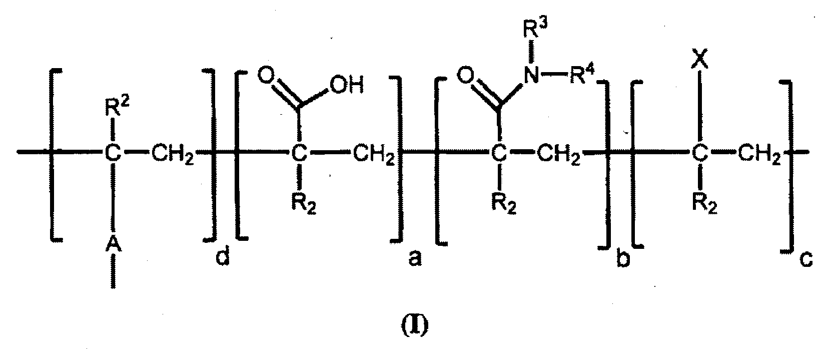 wobei
a wenigstens eine Acryloylcarboxylsäuregruppe umfasst,
b wenigstens eine Acryloylamidgruppe umfasst,
c wenigstens eine reaktive Gruppe X umfasst, die zur Immobilisierung eines Biogegenstandes geeignet ist und
d wenigstens einen Haftvermittler an der Oberflächenbefestigungsgruppe A umfasst,
wobei
A eine an der Substratoberfläche befestigte Haftvermittlergruppe ist und mit den Acryloylmonomeren copolymerisiert ist,
**R²** H oder ein (C₁₋₄)-Alkyl ist,
und **R³** und **R⁴** jeweils unabhängig voneinander H oder ein (C₁₋₄)-Alkyl sind,
wobei die Polymerschicht eine Trockendicke von 5 bis 50 Nanometern aufweist.

7. Gegenstand nach Anspruch 6, wobei das Polymer eine Mischung von Acrylsäure- und Acrylamidmonomeren in einem Molverhältnis von 1,0:1,5 bis 1,5:1,0 umfasst und das Polymer ferner einen Quervernetzer in einem Gehalt von 0,001 bis 0,0015 Molprozent basierend auf der Gesamtmolzahl der Monomere umfasst.

8. Gegenstand nach Anspruch 6, wobei die Polymerschicht im wässrigen Medium eine Dicke von 50 bis 300 Nanometern aufweist.

9. Verfahren zur Verwendung des Biosensorgegenstandes nach Anspruch 5, umfassend Berühren der aktivierten Polymerschicht mit einem Biogegenstand, um wenigstens einen Biogegenstand zu absorbieren, zu immobilisieren oder einer Kombination davon.

10. Verfahren nach Anspruch 9, wobei der Biosensor einen Resonanzwellenleiterbiosensor ist und eine Biogegenstandsimmobilisierungsantwort von 1.500 pm oder mehr bereitstellt.

11. Verfahren nach Anspruch 9, ferner umfassend Berühren des absorbierten oder immobilisierten Biogegenstandes mit einem Kandidatenliganden und Detektieren einer Interaktion zwischen dem Biogegenstand und dem Liganden.

12. Verfahren nach Anspruch 11, wobei die detektierte Interaktion zwischen dem Biogegenstand und dem Liganden mit dem Biosensor zwischen 20 und 200 pm beträgt.

13. Verfahren nach Anspruch 11, wobei 20 Gewichts-% oder mehr des immobilisierten Biogegenstandes mit einem Liganden binden.

14. Gegenstand nach Anspruch 6, wobei der Biosensor vom Resonanzwellenleitertyp ist.

15. Gegenstand nach Anspruch 5, wobei die reaktive Gruppe ein Sulfo-NHS-Ester ist.

## Revendications

1. Article biocapteur sans marqueur comprenant :
un substrat ;
une couche de liaison fixée à la surface du substrat ; et
une couche de copolymère acrylique-acrylamide fixée à la couche de liaison ;
où la couche de copolymère acrylique-acrylamide a une épaisseur sèche de 1 à 100 nanomètres, et
où le copolymère acrylique-acrylamide comprend un mélange de monomères de :
au moins un acide acrylique ou un acide alkylacrylique, ou une combinaison de ceux-ci ; et
au moins un acrylamide, acrylamide N-substitué, acrylamide N,N'-disubstitué, alkylacrylamide, alkylacrylamide N-substitué ou alkylacrylamide N,N'-disubstitué, ou une combinaison de ceux-ci.

2. Article selon la revendication 1 où le substrat comprend au moins l'un d'un plastique, une substance polymérique ou copolymérique, une céramique, un verre, un composite, un matériau cristallin, un métal, un métal noble, un métal semi-noble, un oxyde métallique, un oxyde non-métallique, un métal de transition ou une combinaison de ceux-ci.

3. Article selon la revendication 1 où le copolymère acrylique-acrylamide comprend un mélange d'au moins l'un des monomères acides acryliques et d'au moins l'un des monomères acrylamides dans un rapport molaire de 1,0:2,0 à 2,0:1,0.

4. Article selon la revendication 1 tel que le copolymère acrylique-acrylamide est réticulé, le réticulant étant présent en une quantité de 0,0001 à 0,0015 mol % sur la base des moles totales de monomère.

5. Article selon la revendication 1 tel que le copolymère acrylique-acrylamide a au moins un groupe réactif approprié pour immobiliser une bioentité.

6. Article biocapteur comprenant :
un substrat ayant une couche de liaison fixée à la surface du substrat, et
une couche de polymère fixée à la couche de liaison, le polymère comprenant des mères de formule (I) : 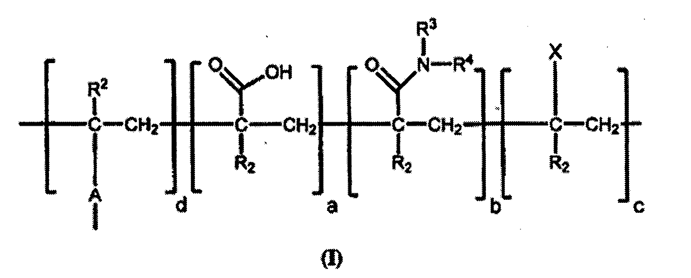 où :
***a*** comprend au moins un groupe acide acryloylcarboxylique ;
***b*** comprend au moins un groupe acryloylamide ;
***c*** comprend au moins un groupe réactif **X** approprié pour immobiliser une bioentité ; et
***d*** comprend au moins un groupe **A** de fixation de la couche de liaison à la surface,
où
***A*** est un groupe de couche de liaison fixé à la surface du substrat et copolymérisé avec les monomères acryloyle ;
**R²** est H ou (C₁₋₄)alkyle ; et
**R³** et **R⁴** sont chacun indépendamment H ou (C₁₋₄)alkyle,
la couche de polymère ayant une épaisseur sèche de 5 à 50 nanomètres.

7. Article selon la revendication 6 où le polymère comprend un mélange de monomères acides acryliques et acrylamides dans un rapport molaire de 1,0:1,5 à 1,5:1,0, et le polymère comprend en outre un réticulant en une quantité de 0,001 à 0,0015 mol % sur la base des moles totales de monomère.

8. Article selon la revendication 6, où la couche de polymère en milieu aqueux a une épaisseur de 50 à 300 nanomètres.

9. Procédé d'utilisation de l'article biocapteur selon la revendication 5 comprenant la mise en contact de la couche de polymère activée avec une bioentité pour absorber, immobiliser, ou une combinaison de ceux-ci, au moins une bioentité.

10. Procédé selon la revendication 9 où le biocapteur est un biocapteur guide d'onde à résonance et fournit une réponse d'immobilisation de bioentité de 1 500 pm ou plus.

11. Procédé selon la revendication 9 comprenant en outre la mise en contact de la bioentité absorbée ou immobilisée avec un ligand candidat et la détection d'une interaction entre la bioentité et le ligand.

12. Procédé selon la revendication 11 où l'interaction détectée entre la bioentité et le ligand avec le biocapteur est de 20 à 200 pm.

13. Procédé selon la revendication 11 où 20 % en poids ou plus de la bioentité immobilisée se lient avec un ligand.

14. Article selon la revendication 6 où le biocapteur est un type guide d'onde résonant.

15. Article selon la revendication 5 où le groupe réactif est un ester de sulfo-NHS.
